(19) Europäisches Patentamt
European Patent Office
Office européen des brevets

(11) Numéro de publication: **0 223 671 B1**

(12) **FASCICULE DE BREVET EUROPEEN**

(45) Date de publication de fascicule du brevet: **27.11.91**

(51) Int. Cl.[5]: **A61K 31/57**, //(A61K31/57, 31:135)

(21) Numéro de dépôt: **86402328.8**

(22) Date de dépôt: **16.10.86**

(54) **Médicament à action synergétique anti-inflammatoire à base d'un corticostéroïde et d'un beta-agoniste.**

(30) Priorité: **16.10.85 FR 8515355**

(43) Date de publication de la demande: **27.05.87 Bulletin 87/22**

(45) Mention de la délivrance du brevet: **27.11.91 Bulletin 91/48**

(84) Etats contractants désignés: **BE CH DE FR GB IT LI NL SE**

(56) Documents cités: **GB-A- 2 107 715**

**UNLISTED DRUGS, vol. 33, no. 6, juin 1981, page 101c, Albert J. PHIEBIG INC., New York, US; "Ventolin compositum"**

(73) Titulaire: **CENTRE INTERNATIONAL DE RECHERCHES DERMATOLOGIQUES GALDERMA - CIRD GALDERMA**
**Sophia Antipolis**
**F-06560 Valbonne(FR)**

(72) Inventeur: **Shroot, Braham Villa 35**
**Hameaux de Val Bosquet Chemin de Val Bosquet**
**F-06600 Antibes(FR)**
Inventeur: **Hensby, Christopher**
**89, Chemin d'Andon Villa Madru**
**F-06410 Biot(FR)**

(74) Mandataire: **Nony, Michel et al**
**Cabinet NONY & CIE, 29, rue Cambacérès**
**F-75008 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

## Description

La présente invention a pour objet un médicament à action synergétique anti-inflammatoire associant d'une part, un corticostéroïde et d'autre part, un $\beta$-agoniste.

Le médicament selon l'invention trouve tout particulièrement une application dans le traitement et la prévention des réactions inflammatoires rencontrées lors de dermatites et dermatoses diverses (atopique, eczématiforme, exfoliative, de contact, séborrhéique), lors de psoriasis, d'hyperkératose épidermolytique, d'ichthyosis lamellaire, de pemphigus, goutte et autres inflammations de la peau pouvant être provoquées par des agents chimiques ou mécaniques, par des chocs thermiques ou par des radiations.

De plus, le médicament selon l'invention peut être utilisé dans le domaine vétérinaire, par exemple dans le traitement de maladies de la peau caractérisées par une inflammation et une dermite (dermite exsudative ou sèche, dermite eczémateuse, dermite de contact, dermite séborrhéique, dermite suite à une infestation parasitaire).

Les corticostéroïdes constituent une classe d'anti-inflammatoires présentant une bonne activité thérapeutique à l'égard de nombreuses maladies inflammatoires de la peau. Toutefois, un usage prolongé provoque de nombreux inconvénients, plus particulièrement une atrophie de la peau. Par ailleurs, lors de l'interruption du traitement par les corticostéroïdes, les symptômes inflammatoires ont tendance à réapparaître sous forme plus aigüe.

En vue de pallier aux effets secondaires des corticostéroïdes, d'autres substances ont été proposées aux dermatologues, notamment des inhibiteurs de la synthétase prostaglandine, mais ces composés sont peu actifs à l'égard des dermatoses à caractère inflammatoire chronique.

Par ailleurs, certains agonistes adrénergiques plus particulièrement du type $\beta_2$ sélectif dont le Salbutamol (INN) ont été décrits comme ayant une activité anti-inflammatoire par voie topique.

Les agonistes $\beta_2$ sélectifs étaient, préalablement à la mise en évidence de ces. propriétés anti-inflammatoires, plus connus comme étant susceptibles de réagir sélectivement avec les récepteurs $\beta_2$ présents dans les muscles lisses des vaisseaux sanguins et des bronches, déclenchant ainsi une dilatation des bronches et des vaisseaux.

Les agonistes $\beta_2$ se différencient des agonistes $\beta_1$ dans la mesure où ces derniers réagissent de façon sélective avec les récepteurs $\beta_1$ et déclenchent une stimulation cardiaque.

Les agonistes $\beta_2$ sélectifs ne sont pas néanmoins dénués d'une activité cardio-vasculaire, de telle sorte qu'à certaines concentrations par application topique, l'action anti-inflammatoire peut entraîner, par voie d'absorption rapide de l'épiderme, une tachycardie sinusale ainsi que d'autres effets indésirables tels qu'agitations, tremblements ou vertiges.

Ces effets secondaires, bien que différents de ceux des corticostéroïdes, ont néanmoins considérablement freiné l'utilisation de ces substances comme anti-inflammatoires par voie externe.

Après de nombreuses recherches sur ces deux classes d'anti-inflammatoires, on a maintenant constaté de façon tout à fait surprenante qu'un effet de synergie particulièrement prononcé pouvait être obtenu en associant au moins un corticostéroïde à une concentration à laquelle il ne provoque pas les effets secondaires connus des corticostéroïdes, et un $\beta$-agoniste.

En associant ainsi un agoniste $\beta$ à un corticostéroïde, on a noté une augmentation particulièrement significative de l'activité anti-inflammatoire sans rencontrer par ailleurs les effets secondaires propres aux $\beta$-agonistes, notamment une tachycardie.

La présente invention a donc pour objet un médicament à action synergétique anti-inflammatoire pour une application sur la peau caractérisé par le fait qu'il contient en association, dans un véhicule pharmaceutique acceptable pour une administration sur la peau, au moins un corticostéroïde en une proportion comprise entre 0,0005 et 0,5% en poids et au moins un $\beta$-agoniste en une proportion comprise entre 0.05 et 5 %, le $\beta$-agoniste étant un des dérivés de phénéthanolamine ayant une activité anti-inflammatoire et correspondant à la formule générale suivante :

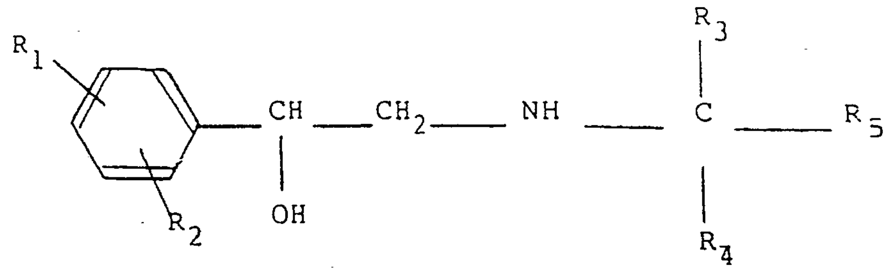

dans laquelle :

| | |
|---|---|
| $R_1$ et/ou $R_2$ | identiques ou différents, représentent OH, $-CH_2-OH$, $-OCO-(C_6H_4)-CH_3$, ou $-NH-SO_2-CH_3$ |
| $R_3$ et $R_4$ | représentent indépendamment l'un de l'autre un atome d'hydrogène ou un groupe alkyle inférieur ayant de 1 à 4 atomes de carbone, $R_3$ et $R_4$ ne pouvant simultanément représenter un atome d'hydrogène, |
| $R_5$ | représente un radical alkyle, linéaire ou ramifié, ayant de 1 à 4 atomes de carbone, éventuellement interrompu par un atome d'oxygène, un radical aryle, un radical aralkyle ou un radical aralkyloxy, et les sels des acides minéraux ou organiques desdits composés notamment les chlorhydrates et les sulfates. |

L' expression "application sur la peau" telle qu'utilisée selon l'invention signifie une application locale non seulement sur la surface de la peau du corps mais également sur la surface du cuir chevelu et des ongles sans que de préférence aucun effet systémique simultané ne se produise.

L'effet de synergie de l'association corticostéroïde/$\beta$-agoniste a pu être mise en évidence à l'aide des tests d'évaluation des effets anti-inflammatoires connus à savoir d'une part, le test de l'oedème je l'oreille induit par l'huile de croton chez le rat (C. TONELLI; L. THIBAULT and I. RINGLER "A Bio-Assay for the Concomitant Assessment of the Anaphlogistic and Thymolytic Activities of Topically Applied Corticoits", Endocrinology; 1965; 77; 625-634 et J.C Le DOUAREC et M. VISALLI "De l'Etude de l'Action Locale des Anti-Inflammatoires" J. Pharmacologie (Paris), 1970; 1; 3; 395-405) et d'autre part, le test de l'érythème induit par UVB chez le cobaye (M.L GRAEME et al. "The Effect of Topically Applied Agents on JV Erythema in Guinea Pigs". Pharmacologist 1975. 17, 281).

En outre, on a pu montrer que lorsque l'association contenait un bloquant adrénergique tel que le propanolol, on n'observait plus aucun effet anti-inflammatoire, ce qui démontre bien qu'il y a effectivement effet de synergie entre d'une part le corticostéroïde et d'autre part l'agoniste $\beta_2$.

Les corticostéroïdes de la composition synergétique selon l'invention peuvent être l'un quelconque des stéroïdes anti-inflammatoires connus ainsi que des mélanges de ceux-ci. On peut citer en particulier sans que cette énumération soit limitative, les suivants :

Betaméthasone-17 benzoate,
Betaméthasone-17 dipropionate,
Betaméthasone-17 valérate,
Clobétasol-17 propionate,
Clobétasone-17 butyrate,
Désonide,
Desoxyméthasone,
Dexaméthasone,
Diflucortolone valérate,
Diflorasone diacétate,
Fluméthasone pivalate,
Fluclorolone acétonide,
Flucortin butylester,
Fluocinolone acétonide,
Fluocortolone,
Fluprédnidène (fluprédnylidène) acétate,
Flurandrénolone,
Halcinonide,

Hydrocortisone + urée
Hydrocortisone (alcool ou acétate),
Hydrocortisone-17 butyrate,
Méthylprédnisolone,
Triamcinolone acétonide,

A cette énumération, on peut également ajouter les orthoesters d'hydrocortisone tels que décrits dans le Brevet U.S.-A-4.264.584

Parmi les corticostéroïdes ayant donné les résultats les plus significatifs, on peut tout particulièrement citer :

- l'Hydrocortisone,
- l'Hydrocortisone-17 butyrate,
- la Betaméthasone-17 valérate, et
- le Clobétasol-17 propionate.

Dans la composition synergétique selon l'invention, la concentration en corticostéroïde dépend bien entendu de son activité propre et l'on conçoit que cette concentration puisse varier dans de larges limites mais est toutefois généralement comprise entre 0,0005 et 0,5 % en poids et de préférence comprise entre 0,001 et 0,2 %.

Parmi les $\beta$-agonistes utilisables selon l'invention, on peut citer le Salbutamol et ses dérivés, l'Isoprenaline et ses dérivés, la Terbutaline et ses dérivés ainsi que les composés décrits dans le brevet britannique 2.140.800 et dans la demande de brevet français 84.03020 FR-A-2.541.999.

Parmi les $\beta$-agonistes particulièrement préférés ayant donté d'excellents résultats avec les corticostéroïdes mentionnés ci-dessus, on doit tout particulièrement mentionner :

- le Salbutamol ou 2-(tert-butylamino)-1-(4-hydroxy-3-hydroxyphényl)éthanol, et son sulfate,
- la Terbutaline ou 1-(3,5-dihydroxyphényl)-2-(tert-butylamino)éthanol et son sulfate, et
- l'Isoprénaline ou 1-(3,4-dihydroxyphényl)-2-isopropylamino éthanol et son chlorhydrate.

La concentration en $\beta$-agoniste peut, comme indiqué ci-dessus, varier dans de larges limites mais celles-ci en fonction notamment des composés préférés est de préférence entre 0,1 et 3 % en poids.

De préférence le rapport en poids du corticostéroïde au $\beta$-agoniste est compris entre 1:1 à 1:100, de préférence entre 1:1 et 1:50.

Parmi les associations d'un corticostéroïde et d'un $\beta$-agoniste ayant conduit à une synergie importante, on peut notamment citer les suivantes :

. Hydrocortisone/Salbutamol base,
. Hydrocortisone/Salbutamol Sulfate,
. Hydrocortisone-17 Butyrate/Salbutamol base,
. Betaméthasone-17 Valérate/Salbutamol base,
. Clobétasol-17 Propionate/Salbutamol base,
. Hydrocortisone/Terbutaline Sulfate,
. Hydrocortisone-17 butyrate/Terbutaline Sulfate
. Hydrocortisone-Isoprenaline chlorhydrate

Le médicament selon l'invention peut se présenter sous différentes formes, notamment sous forme d'une pommade, d'une émulsion, d'une lotion, ou d'un gel, ou sous d'autres formes pharmaceutiquement acceptables pour une application sur la peau.

Le terme "pommade" couvre des formulations de type anhydre constituées par exemple de vaseline (ou autre hydrocarbure), d'huiles, d'esters à chaîne grasse, de glycérides, d'acides gras ou d'alcool gras, de lanoline, de polyéthylèneglycols ainsi que leurs dérivés et leurs mélanges.

Ces pommades peuvent également contenir des dérivés amphiphiles conférant à la formule une certaine affinité pour l'eau.

Ces pommades peuvent être préparées par dispersion des ingrédients actifs dans la base convenable huileuse.

Les émulsions sont préparées en dispersant ou en solubilisant les composés actifs en fonction de leur affinité, soit dans la phase grasse, sait dans la phase aqueuse soit dans les deux phases avant la mise en émulsion. La phase huileuse peut contenir des corps gras d'origine minérale, végétale ou animale ainsi que des silicones, des alcools gras, acides gras ou esters à chaîne grasse, notamment du palmitate d'isopropyle, du myristate d'isopropyle, de butyle et de cétyle, du stéarate d'hexadécyle, du palmitate d'éthyle, des triglycérides comme par exemple les glycérides des acides octanoïque et décanoïque ainsi que du ricinoléate de cétyle.

La phase aqueuse peut contenir des polyols tels que le propylène glycol ou le glycérol.

L'agent émulsionnant peut être de nature non ionique, par exemple ester ou alcool gras oxyéthyléné, ou

anionique par exemple un alkylsulfate de sodium ou de potassium.

Les lotions sont des solutions ou des dispersions des composés actifs dans un support approprié.

Comme support approprié, on peut citer les alcools aliphatiques tels que l'éthanol ou l'isopropanol, les polyols tels que la glycérine, les polyalkylèneglycols ; le monoéthyléther du diéthylène glycol; les solvants organiques compatibles avec la peau ainsi que l'eau et les mélanges de ceux-ci.

Les gels sont des préparations semi-solides préparés par épaississement d'une solution ou d'une suspension des ingrédients actifs, à l'aide d'agents gélifiants organiques ou minéraux tels que les "bentones", la silice, les polyéthylènes micronisés ou l'acide polyacrylique réticulé, les dérivés de cellulose ou les gommes d'origine naturelle ou synthétique.

On peut également, si on le souhaite, afin de faciliter une meilleure dispersion et disponibilité des principes actifs, incorporer un alcool aliphatique inférieur tel que l'éthanol et éventuellement une huile de silicone, des glycols tels que des polypropylène ou polyéthylène glycols.

Dans le traitement des dermatites et dermatoses, le médicament synergétique anti-inflammatoire selon l'invention est appliqué au moins une fois par jour sur les lésions à traiter à raison de 0,5 à 10 mg/cm$^2$, la durée du traitement pouvant être de l'ordre de 2 à 6 semaines suivant les zones de peau à traiter et l'intensité de l'inflammation.

EVALUATION DE L'EFFET DE SYNERGIE

I - TEST DE L'OEDEME DE L'OREILLE PROVOQUE PAR L'HUILE DE CROTON CHEZ LE RAT

On applique de façon normalisée sur les faces interne et externe de l'oreille droite de rats une solution d'huile de croton ayant la composition suivante:

- pyridine : 4ml
- oxyde de diéthyle: 13ml
- huile de croton *: 2ml
- eau qsp 20ml

Les animaux traités reçoivent, une heure avant l'application de l'huile de croton, une application sur l'oreille droite par voie topique, du produit étudié dans le véhicule suivant (placebo):gel hydroéthanolique à 2% d'hydroxypropyl cellulose (KLUCEL HF); (proportion eau-éthanol : 80/20).

Les animaux témoins ne reçoivent que l'application d'huile de croton.

Six heures après l'application d'huile de croton, on évalue l'importance de l'oedème par comparaison de l'oreille droite et de l'oreille gauche. On calcule ensuite l'inhibition de l'oedème, en pourcentage, par rapport aux animaux témoins.

* SIGMA réf. C4755

TABLEAU A

| | DOSE (% p/v) | POURCENTAGE D'INHIBITION | STATISTIQUE TEST DE "T" DE STUDENT (a) |
|---|---|---|---|
| 1/ PLACEBO VEHICULE (b) | 0 | 10 | NS |
| 2/ PLACEBO plus | | | |
| .HYDROCORTISONE | 0.1 | 14 | NS |
| 3/ PLACEBO plus | | | |
| .SALBUTAMOL SULFATE | 0.5 | 14 | NS |
| 4/ PLACEBO plus | | | |
| .PROPRANOLOL | 0.5 | 5 | NS |
| 5/ PLACEBO plus | | | |
| .HYDROCORTISONE | 0.1 | | |
| .SALBUTAMOL SULFATE | 0.5 | 24 | ** |
| 6/ PLACEBO plus | | | |
| .HYDROCORTISONE | 0.1 | | |
| .SALBUTAMOL SULFATE | 0.5 | | |
| .PROPRANOLOL | 0.5 | 14 | NS |
| 7/ PLACEBO plus | | | |
| .TERBUTALINE SULFATE | 0.5 | 12 | NS |
| 8/ PLACEBO plus | | | |
| .HYDROCORTISONE | 0.1 | | |
| .TERBUTALINE SULFATE | 0.5 | 48 | ** |
| 9/ PLACEBO plus | | | |
| .HYDROCORTISONE 17 BUTYRATE | 0.1 | 30 | * |
| 10/ PLACEBO plus | | | |
| .HYDROCORTISONE 17 BUTYRATE | 0.1 | | |
| .SALBUTAMOL | 0.5 | 59 | *** |

(a) NS = NON SIGNIFICATIF $P > 0.05$
* = $P < 0.05$ $P > 0.01$
** = $P < 0.01$ $P > 0.001$
*** = $P < 0.001$

(b) PLACEBO VEHICULE = GEL HYDRO-ETHANOLIQUE 80/20 EN VOLUME AU KLUCEL 2%.

## II - TEST DE L'ERYTHEME INDUIT PAR UVB CHEZ LE COBAYE

Les flancs des cobayes sont rasés à la tondeuse 24 heures avant l'expérience. On soumet les animaux à une irradiation à l'aide d'une lampe à vapeur de mercure. On irradie pendant 30 secondes deux sites de

diamètre 1,5cm sur chaque flanc. Une heure après l'irradiation, on applique par voie topique le produit étudié sur chaque site irradié d'un seul flanc. Les observations de l'érythème sont faites 2h, 3,5h, 5h et 24h après l'application du produit. L'érythème est évalué selon une échelle de 0 à 5.

Les produits sont appliqués dans le véhicule suivant:

N,N-diméthylacétamide: 20ml
acétone : 40ml
éthanol : 40ml

On applique un volume de 100 microlitres par site.

On évalue l'importance de l'érythème en comparant les flancs droit et gauche. On calcule ensuite l'inhibition de l'érythème, en pourcentage, par rapport aux animaux témoins ayant reçu une application du véhicule seul.

TABLEAU B

| | DOSE (% p/v) | POURCENTAGE D'INHIBITION DE L'ERYTHEME 5 HEURES APRES APPLICATION (STATISTIQUE TEST DE "T" DE STUDENT) (a) | |
|---|---|---|---|
| 1/ PLACEBO VEHICULE (b) | 0 | 5 | NS |
| 2/ PLACEBO plus .HYDROCORTISONE | 5 | 3 | NS |
| 3/ PLACEBO plus .SALBUTAMOL | 5 | 14 | * |
| 4/ PLACEBO plus .HYDROCORTISONE .SALBUTAMOL | 5<br>5 | 56 | *** |

Pour (a) et (b) voir tableau A

On va maintenant donner à titre d'illustration plusieurs exemples du médicament synergétique anti-inflammatoire selon l'invention.

## 1. POMMADES

Exemple 1

- Betaméthasone-17 valérate........................ 0,010g
- Salbutamol base.................................. 0,500g
- Huile de vaseline................................ 2,000g
- Paraffine solide................................. 10,000g
- Lanoline hydrogénée.............................. 20,000g
- Conservateurs..................................qs
- Antioxydants...................................qs
- Vaseline.......................................qsp 100,000g

Exemple 2

- Clobétasol-17 propionate......................... 0,010g
- Salbutamol base.................................. 0,500g
- Alcool stéarylique à 20 moles d'oxyde d'éthylène. 40,000g
- Conservateurs..................................qs
- Antioxydants...................................qs
- Huile de ricin.................................qsp 100,000g

Ces pommades peuvent être préparées par dispersion des ingrédients actifs dans la base huileuse convenable.

## 2. EMULSIONS

Exemple 3

- Hydrocortisone-17 butyrate....................... 0,100g
- Terbutaline sulfate.............................. 0,500g
- Alcool cétystéarylique........................... 6,500g
- Alcool cétystéarylique à 20 moles d'oxyde d'éthylène.................................... 2,000g
- Monostéarate de glycérol......................... 2,000g
- Huile de vaseline................................ 15,000g
- Conservateurs..................................qs
- Antioxydants...................................qs
- Eau............................................qsp 100,000g

## 3. LOTIONS

Exemple 4

- Bétaméthasone-17 valérate........................ 0,010g
- Salbutamol base.................................. 0,500g
- Ethanol.......................................... 30,000g
- Antioxydants..................................qs
- PEG 400 Polyéthylène glycol de PM 400............ 100,000g

## 4. ONGUENT

Exemple 5

- Bétaméthasone-17 Valérate........................ 0,01g
- Salbutamol....................................... 0,50g
- Paraffine solide................................. 4,00g
- Huile de vaseline épaisse........................ 2,00g
- Lanoline hydrogénée.............................. 20,00g
- Vaseline blanche................................. 66,99g
- Acide stéarique.................................. 6,00g
- Propylène glycol................................. 0,50g

Un effet thérapeutique a été constaté lorsque cet onguent a été appliqué chez l'homme, une fois par jour, sur une plaque psoriasique.

L'application peut être faite sous occlusion.

## 5. GEL

Exemple 6

- Hydrocortisone................................... 0,100g
- Isoprenaline chlorhydrate........................ 0,500g
- Ethanol.......................................... 20,000g
- Hydroxypropylcellulose........................... 2,000g
- Antioxydants..................................qs
- Eau...........................................qsp 100,000g

6. POMMADE OPHTALMOLOGIQUE

Exemple 7

| | |
|---|---|
| - Hydrocortisone-17 butyrate........................ | 0.01g |
| - Salbutamol base.................................. | 0.05g |
| - Vaseline blanche.................................. | 5.70g |
| - Cholestérine...................................... | 0,30g |
| - Huile d'olive neutralisée et stérile............. | 3,94g |
| - Conservateur...................................... | q.s |
| - Antioxydant....................................... | q.s |

Les pommades ophtalmologiques sont laminées au broyeur à cylindre de marbre ou de porphyre et passées aux homogénéiseurs les plus fins. Les principes actifs sont préalablement micronisés par trituration avec la base fondue. Les pommades ophtalmologiques sont conditionnées dans des tubes à embout très mince, soigneusement obturés, évitant toute contamination. Ces tubes sont stérilisés au préalable. Les principes actifs, excipients et conditionnement, doivent être stériles.

**Revendications**

**1.** Médicament à action synergétique anti-inflammatoire pour une application sur la peau, caractérisé par le fait qu'il contient en association, dans un véhicule pharmaceutique acceptable pour une administration sur la peau, au moins un corticostéroïde en une proportion comprise entre 0,0005 et 0,5% en poids et au moins un $\beta$-agoniste en une proportion comprise entre 0,05 et 5 %, le dit $\beta$-agoniste étant un dérivé de phénéthanolamine correspondant à la formule générale suivante :

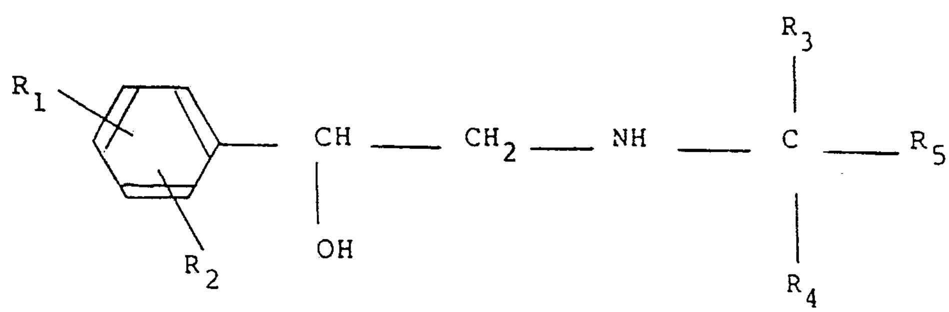

dans laquelle :

$R_1$ et/ou $R_2$ identiques ou différents, représentent OH $-CH_2-OH$ - $OCO\{C_6H_4\}CH_3$ ou - NH - $SO_2$ $-CH_3$,

$R_3$ et $R_4$ , représentent, indépendamment l'un de l'autre, un atome d'hydrogène ou un groupe alkyle inférieur ayant de 1 à 4 atomes de carbone, $R_3$ et $R_4$ ne pouvant simultanément représenter un atome d'hydrogène,

$R_5$ représente un radical alkyle, linéaire ou ramifié, ayant de 1 à 4 atomes de carbone, éventuellement interrompu par un atome d'oxygène, un radical aryle, un radical aralkyle ou un radical aralkyloxy,

et les sels des acides minéraux ou organiques desdits composés notamment les chlorhydrates et les sulfates.

**2.** Médicament selon la revendication 1, caractérisé par le fait que le corticostéroïde est l'hydrocortisone, l'hydrocortisone-17 butyrate, la Betaméthasone-17 valérate ou le Clobétasol-17 propionate.

**3.** Médicament selon la revendication 1 ou 2, caractérisé par le fait que la concentration en corticostéroïde

est de préférence comprise entre 0,001 et 0,2 % en poids.

4. Médicament selon l'une quelconque des revendications précédentes, caractérisée par le fait que le $\beta$-agoniste est le Salbutamol ou son sulfate, la Terbutaline ou son sulfate, ou l'Isoprenaline ou son chlorhydrate.

5. Médicament selon l'une quelconque des revendications précédentes, caractérisé par le fait que la concentration en $\beta$-agoniste est de préférence comprise entre 0,1 et 3%.

6. Médicament selon l'une quelconque des revendications précédentes, caractérisé par le fait que le rapport en poids du corticostéroïde au $\beta$-agoniste est de préférence compris entre 1:1 à 1:100, de préférence entre 1:1 et 1:50.

7. Médicament selon l'une quelconque des revendications précédentes, caractérisé par le fait que l'association corticostéroïde)/$\beta$-agoniste est de préférence prise dans le groupe constitué par les associations suivantes :
   - Hydrocortisone/Salbutamol base,
   - Hydrocortisone/Salbutamol sulfate,
   - Hydrocortisone-17 butyrate/Salbutamol base,
   - Betaméthasone-17 valérate/Salbutamol base,
   - Clobétasol-17 propionate/Salbutamol base,
   - Hydrocortisone/Terbutaline Sulfate,
   - Hydrocortisone-17 butyrate/Terbutaline sulfate et
   - Hydrocortisone/Isoprénaline chlorhydrate.

8. Composition synergétique anti-inflammatoire pour une application sur la peau sous forme d'une pommage, d'une émulsion, d'un gel ou d'une lotion, caractérisée par le fait qu'elle contient en association au moins un corticostéroïde et au moins un $\beta$-agoniste, ledit corticostéroïde étant présent à une concentration comprise entre 0,0005 et 0,5% en poids.

9. Composition selon la revendication 8, caractérisée par le fait que la concentration en $\beta$-agoniste est comprise entre 0,05 et 5% en poids et de préférence entre 0,1 et 3%.

10. Composition selon l'une quelconque des revendications 8 et 9 caractérisée par le fait qu'elle contient un alcool aliphatique inférieur, une huile de silicone ou un glycol en vue d'une meilleure disponibilité des principes actifs.

11. Utilisation de l'association d'au moins un corticostéroïde et d'au moins un $\beta$-agoniste pour la préparation d'une composition pharmaceutique à action synergétique locale contenant dans un véhicule approprié pour une application sur la peau, ledit corticostéroïde à une concentration comprise entre 0,0005 et 0,5% en poids et ledit $\beta$-agoniste à une concentration comprise entre 0,05 et 5% en poids, ladite composition étant destinée au traitement des inflammations de la peau.

12. Utilisation selon la revendication 11, caractérisée par le fait que ladite composition est destinée au traitement des dermatites et dermatoses, du psoriasis, des hyperkératoses épidermolytiques, d'ichtyosis lamellaire, du pemphigus ou de la goutte.

**Claims**

1. Medicinal product having an anti-inflammatory synergistic action for application to the skin, characterised in that it contains, in combination, in a pharmaceutical vehicle acceptable for administration to the skin, at least one corticosteroid in a proportion of between 0.0005 and 0.5% by weight and at least one $\beta$-agonist in a proportion of between 0.05 add 5%, the said $\beta$-agonist being a phenethanolamine derivative corresponding to the following general formula:

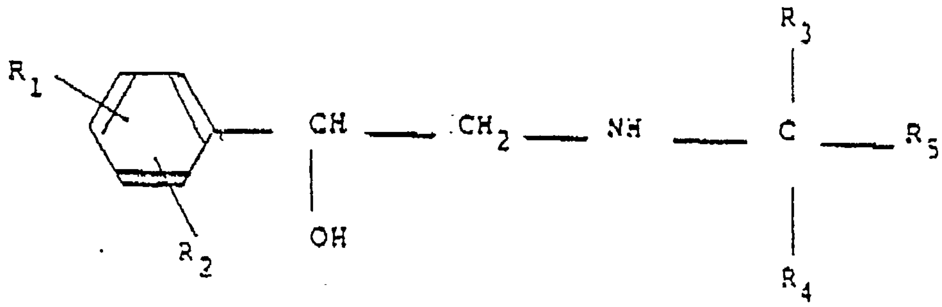

in which:

$R_1$, and/or $R_2$, which may be identical or different, represent OH, $-CH_2-OH$, $-OCO(C_6H_4)CH_3$ or $-NH-SO_2-CH_3$,

$R_3$ and $R_4$, represent, independently of one another, a hydrogen atom or a lower alkyl group having from 1 to 4 carbon atoms, it not being possible for $R_3$ and $R_4$ simultaneously to represent a hydrogen atom,

$R_5$ represents a linear or branched alkyl radical having from 1 to 4 carbon atoms, optionally interrupted by an oxygen atom, an aryl radical, an aralkyl radical or an aralkyloxy radical,

and the salts of the said compounds with inorganic or organic acids, in particular the hydrochlorides and the sulphates.

2. Medicinal product according to Claim 1, characterised in that the corticosteroid is hydrocortisone, hydrocortisone 17-butyrate, betamethasone 17-valerate or clobetasol 17-propionate.

3. Medicinal product according to Claim 1 or 2, characterised in that the concentration of cortico-steroid is preferably between 0.001% and 0.2% by weight.

4. Medicinal product according to any one of the preceding claims, characterised in that the β-agonist is salbutamol or its sulphate, terbutaline or its sulphate or isoprenaline or its hydrochloride.

5. Medicinal product according to any one of the preceding claims, characterised in that the concentration of β-agonist is preferably between 0.1 and 3%.

6. Medicinal product according to any one of the preceding claims, characterised in that the weight ratio of the corticosteroid to the β-agonist is preferably between 1:1 and 1:100, preferably between 1:1 and 1:50.

7. Medicinal product according to any one of the preceding claims, characterised in that the corticosteroid/β-agonist combination is preferably selected from the group consisting of the following combinations:
   - hydrocortisone/salbutamol base,
   - hydrocortisone/salbutamol sulphate,
   - hydrocortisone 17-butyrate/salbutamol base,
   - betamethasone 17-valerate/salbutamol base,
   - clobetasol 17-propionate/salbutamol base,
   - hydrocortisone/terbutaline sulphate,
   - hydrocortisone 17-butyrate/terbutaline sulphate,
   and
   - hydrocortisone/isoprenaline hydrochloride.

8. Synergistic anti-inflammatory composition for application to the skin in the form of an ointment, an emulsion, a gel or a lotion, characterised in that it contains, in combination, at least one corticosteroid and at least one β-agonist, the said corticosteroid being present at a concentration of between 0.0005 and 0.5% by weight.

9. Composition according to Claim 8, characterised in that the concentration of $\beta$-agonist is between 0.05 and 5% by weight, and preferably between 0.1 and 3%.

10. Composition according to either of Claims 8 and 9, characterised in that it contains a lower aliphatic alcohol, a silicone oil or a glycol for the purpose of enhancing the availability of the active principles.

11. Use of the combination of at least one corticosteroid and at least one $\beta$-agonist far the preparation of a pharmaceutical composition having a local synergistic action and containing, in a vehicle suitable for application to the skin, the said corticosteroid at a concentration of between 0.0005 and 0.5% by weight and the said $\beta$-agonist at a concentration of between 0.05 and 5% by weight, the said composition being intended for the treatment of skin inflammations.

12. Use according to Claim 11, characterised in that the said composition is intended for the treatment of dermatitis and dermatosis, psoriasis, epidermolytic hyperkeratosis, lamellar ichthyosis, pemphigus or gout.

**Patentansprüche**

1. Antiinflammatorisches Arzneimittel mit synergistischer Wirkung zum Auftragen auf die Haut

**dadurch gekennzeichnet,** daß

es in einem pharmazeutisch verträglichen, auf die Haut aufzutragenden Träger mindestens ein Corticosteroid in einer Menge von 0,0005 und 0,5 Gew.-% in Kombination mit mindestens einem $\beta$-Agonisten in einer Menge von 0,05 bis 5 Gew.-% enthält, wobei der $\beta$-Agonist ein Phenethanolamin-Derivat der folgenden allgemeinen Formel:

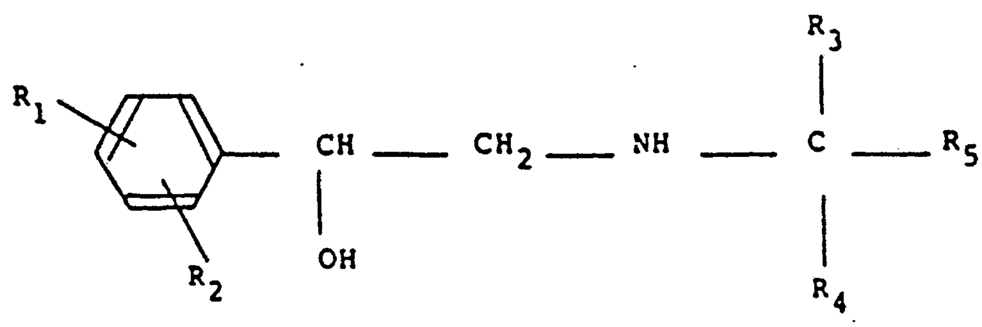

worin

| | |
|---|---|
| $R_1$ und/oder $R_2$, | die gleich oder verschieden sind, für OH, $-CH_2-OH$, - $OCO-(C_6H_4)-CH_3$ oder - $NH-SO_2-CH_3$ stehen, |
| $R_3$ und $R_4$ | unabhängig voneinander ein Wasserstoffatom oder eine Niedrig -Alkylgruppe mit 1 bis 4 Kohlenstoffatomen bedeuten, wobei $R_3$ und $R_4$ nicht gleichzeitig ein Wasserstoffatom bedeuten können, und |
| $R_5$ | einen linearen oder verzweigten Alkylrest mit 1 bis 4 Kohlenstoffatomen, der gegebenenfalls durch ein Sauerstoffatom unterbrochen sein kann, einen Arylrest, einen Aralkylrest oder einen Aralkyloxyrest bedeutet, |

oder ein Salz mit einer Mineralsäure oder einer organischen Säure dieser Verbindungen, insbesondere die Hydrochloride und Sulfate, ist.

2. Arzneimittel nach Anspruch 1, dadurch gekennzeichnet, daß es sich bei dem Corticosteroid um Hydrocortison, 17-Hydrocortisonbutyrat, 17-Betamethasonvalerat oder 17-Clobetasolpropionat handelt.

3. Arzneimittel nach Anspruch 1 oder 2, dadurch gekennzeichnet, daß die Konzentration an Corticosteroid vorzugsweise 0,001 bis 0,2 Gew.-% beträgt.

4. Arzneimittel nach einem der vorhergehenden Ansprüche, dadurch gekennzeichnet, daß der $\beta$-Agonist

Salbutamol oder das Sulfat davon, Terbutalin oder das Sulfat davon oder Isoprenalin oder das Hydrochlorid davon ist.

5. Arzneimittel nach einem der vorhergehenden Ansprüche, dadurch gekennzeichnet, daß die Konzentration an β-Agonist vorzugsweise 0,1 bis 3 Gew.-% beträgt.

6. Arzneimittel nach einem der vorhergehenden Ansprüche, dadurch gekennzeichnet, daß das Gewichtsverhältnis von Corticosteroid zu β-Agonist vorzugsweise 1:1 bis 1:100 und insbesondere 1:1 bis 1:50 beträgt.

7. Arzneimittel nach einem der vorhergehenden Ansprüche, dadurch gekennzeichnet, daß die Kombination Corticosteroid/β-Agonist vorzugsweise ausgewählt ist aus der Gruppe bestehend aus folgenden Kombinationen:
   - Hydrocortison/Salbutamolbase,
   - Hydrocortison/Salbutamolsulfat,
   - 17-Hydrocortisonbutyrat/Salbutamolbase,
   - 17-Betamethasonvalerat/Salbutamolbase,
   - 17-Clobetasolpropionat/Salbutamolbase,
   - Hydrocortison/Terbutalinsulfat,
   - 17-Hydrocortisonbutyrat/Terbutalinsulfat und
   - Hydrocortison/Isoprenalinhydrochlorid.

8. Synergistisches anti-inflammatorisches Mittel zum Auftragen auf die Haut in Form einer Pomade, einer Emulsion, eines Gels oder einer Lotion, dadurch gekennzeichnet, daß es mindestens ein Corticosteroid in Kombination mit mindestens einem β-Agonisten enthält, wobei das Corticosteroid in einer Konzentration von 0,0005 bis 0,5 Gew.-% vorhanden ist.

9. Mittel nach Anspruch 8, dadurch gekennzeichnet, daß die Konzentration an β-Agonist 0,05 bis 5 Gew.-% und vorzugsweise 0,1 bis 3 % beträgt.

10. Mittel nach Anspruch 8 und 9,
dadurch gekennzeichnet, daß es einen niedrigen aliphatischen Alkohol, ein Silikonöl oder ein Glykol zur besseren Verfügbarkeit der Wirkstoffe enthält.

11. Verwendung einer Kombination aus mindestens einem Corticosteroid und mindestens einem β-Agonisten zur Herstellung eines pharmazeutischen Mittels mit lokaler synergistischer Wirkung, das in einem zum Auftragen auf die Haut geeigneten Träger das Corticosteroid in einer Konzentration von 0,0005 bis 0,5 Gew.-% und den β-Agonisten in einer Konzentration von 0,05 bis 5 Gew.-% enthält, wobei das Mittel zur Behandlung von Hautentzündungen bestimmt ist.

12. Verwendung nach Anspruch 11, dadurch gekennzeichnet, daß das Mittel zur Behandlung von Dermatitis und Dermatosen, Psoriasis, epidermolytischen Hyperkeratosen, lamellaren Ichtyosen, Pemphigus und Gichtbestimmt ist.